**Europäisches Patentamt**

**European Patent Office**

**Office européen des brevets**

(19)

(11) Numéro de publication : **0 071 242**
**B1**

(12)                 **FASCICULE DE BREVET EUROPÉEN**

(45) Date de publication du fascicule du brevet :
**03.12.86**

(51) Int. Cl.⁴ : **A 61 F   2/30**

(21) Numéro de dépôt : **82106775.8**

(22) Date de dépôt : **27.07.82**

(54) **Tige en titane ou alliage de titane pour prothèse pour fixation sans ciment dans un os long.**

(30) Priorité : **30.07.81 FR 8114841**

(43) Date de publication de la demande :
**09.02.83 Bulletin 83/06**

(45) Mention de la délivrance du brevet :
**03.12.86 Bulletin 86/49**

(84) Etats contractants désignés :
**AT BE CH DE GB IT LI NL SE**

(56) Documents cités :
**FR-A- 2 017 247**
**FR-A- 2 266 491**
**FR-A- 2 310 120**
**FR-A- 2 331 320**
**FR-A- 2 336 913**
**FR-A- 2 429 589**

(73) Titulaire : **CERAVER Société anonyme dite:**
**12, rue de la Baume**
**F-75008 Paris (FR)**

(72) Inventeur : **Blanquaert, Daniel**
**41, rue Dulong**
**D-75017 Paris (FR)**

(74) Mandataire : **Weinmiller, Jürgen et al**
**Lennéstrasse 9**
**D-8133 Feldafing (DE)**

## Description

La présente invention concerne une tige en titane ou alliage de titane pour prothèse articulaire pour fixation sans ciment dans un os long entourée d'un treillis de fil de titane de maille de dimension au moins égale à 0,3 mm, la tige et le treillis étant munis d'un revêtement de protection contre la corrosion à long terme en oxyde de titane.

On connaît une telle tige, notamment par le document FR-A-2 429 589.

Cette tige permet d'obtenir une structure composite treillis-os de module d'élasticité voisin de celui de l'os, exempte de risque de corrosion à long terme et favorisant une repousse sur toute sa longueur.

Toutefois, durant l'implantation de la tige dans la cavité médullaire d'un os long, le treillis peut être détérioré par suite d'un emmanchement à force de la tige qu'il est nécessaire de réaliser pour bloquer celle-ci et éviter tout micro-mouvement par rapport à l'os. Ce blocage est difficile à réaliser, vue la diversité anatomique des os en cause, même si l'on dispose d'une gamme importante de tiges de dimensions différentes.

Il convient donc de protéger le treillis en l'enrobant d'un matériau biocompatible biodégradable.

On connaît, notamment par le document FR-A-2 331 320, une tige entourée d'un ressort à boudin métallique enrobé d'un matériau biocompatible biodégradable. Ce matériau est un dépôt de carbone qui protège bien le treillis lors d'un emmanchement, mais qui n'assure pas une immobilisation totale de la tige après emmanchement, car le module d'élasticité de ce matériau est très éloigné de celui de l'os.

La présente invention a donc pour but de proposer une tige dont le treillis est protégé lors d'un emmanchement par un matériau assurant une immobilisation totale de la tige après emmanchement.

La tige selon l'invention est caractérisée en ce que le treillis est enrobé d'un matériau biocompatible biodégradable de faible module d'élasticité, ce matériau biocompatible biodégradable étant soit un copolymère d'acide lactique et d'acide glycolique, soit du collagène.

Elle répond en outre de préférence à au moins l'une des caractéristiques suivantes :

— Le matériau biocompatible biodégradable est additionné de phosphate tricalcique.

— L'enrobage de matériau biocompatible biodégradable dépasse l'enveloppe du treillis de 0,3 à 1,5 mm.

— Dans une tige pour fixation sans ciment dans la partie supérieure du fémur, le treillis lui est fixé par l'intermédiaire de minces bandes transversales en titane ou alliage de titane, disposées à l'extérieur du treillis métallique et soudées aux surfaces latérales sensiblement planes de la tige sous le rebord d'appui sur la partie supérieure du fémur après résection de sa tête, ainsi que par un point de soudure à la pointe inférieure de la tige.

— Une tige pour fixation sans ciment dans la partie supérieure du fémur est munie sur deux de ses faces opposées d'épaulements disposés dans des plans perpendiculaires à son axe, sur toute sa hauteur jusqu'au rebord d'appui sur la partie supérieure du fémur après résection de sa tête.

Il est décrit ci-après, à titre d'exemple et en référence aux figures du dessin annexé, une tige pour fixation sans ciment dans la partie supérieure du fémur, en vue d'une prothèse de hanche.

La figure 1 représente la tige en élévation latérale du côté des faces comportant les épaulements.

La figure 2 la représente en élévation latérale du côté convexe de l'une des autres faces.

La figure 3 représente une coupe transversale par l'axe III-III de la figure 2.

La tige 1 comporte un rebord 2 d'appui sur la partie supérieure du fémur après sa résection, et un col 3 se terminant par un tenon conique 4 destiné à être implanté dans une tête sphérique (non représentée).

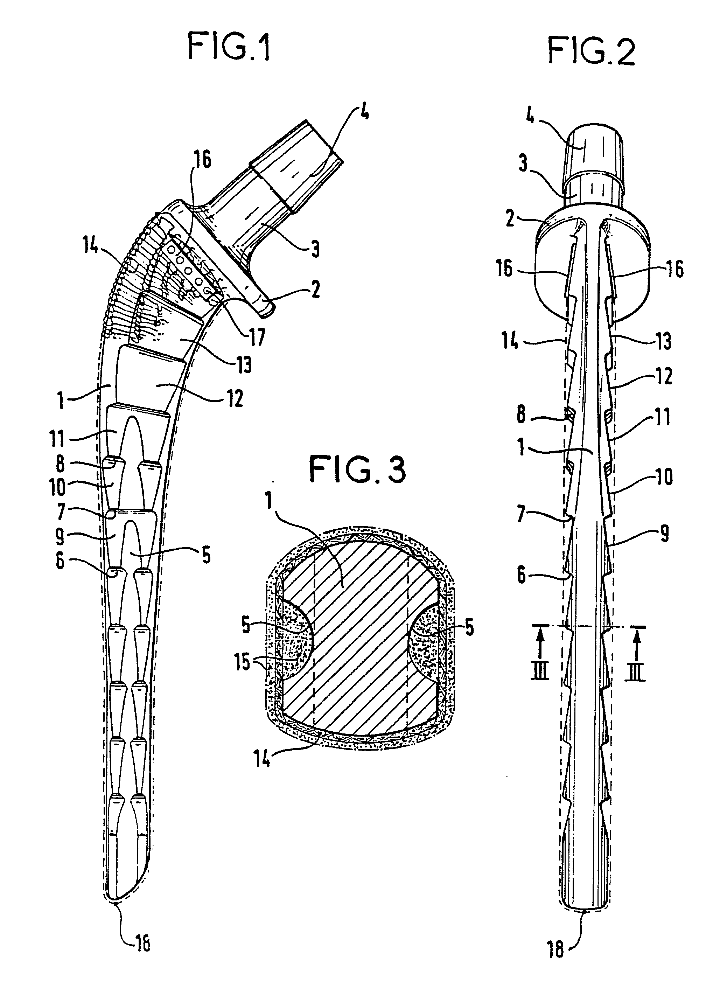

Ses faces latérales vues dans la figure 1 comportent chacune un canal central 5 sur la plus grande partie de leur hauteur, et des épaulements successifs tels que 6, 7, 8 lui donnant l'aspect d'une surface en écailles de poisson doubles 9, 10, 11, puis simples 12, 13. Elle est entourée d'un treillis de fil de titane 14 enrobé d'un copolymère biodégradable d'acide lactique et d'acide glycolique, ou bien de collagène, visible en 15 en figure 3. Ce treillis est tel qu'au cours de la repousse osseuse le module d'élasticité du matériau composite treillis-os naissant soit voisin de celui de l'os existant.

Le treillis de fil de titane est fixé à la partie supérieure de la tige centrale à l'aide de minces barrettes telles que 16, soudées par bombardement électronique en des points de soudure tels que 17. Il est par ailleurs soudé à la pointe inférieure de la tige en un point 18.

La tige et le treillis sont en titane ou en alliage de titane répondant à la désignation TiAl6V4, à 6 % d'aluminium et 4 % de vanadium, et munis d'un revêtement de protection à long terme contre la corrosion en oxyde de titane, obtenu par oxydation anodique.

Sans sortir du cadre de l'invention, la tige peut comporter sur chaque face plusieurs barrettes de fixation du treillis, et le point de soudure du treillis à l'extrémité inférieure de la tige peut éventuellement être remplacée par deux barrettes verticales, disposées sur deux faces opposées. Une tige pour fixation dans un autre os long que le fémur aurait naturellement une forme différente.

## Revendications

1. Tige (1) en titane ou alliage de titane pour prothèse articulaire pour fixation sans ciment dans un os long entourée d'un treillis (14) de fil de titane de maille de dimension au moins égale à 0,3 mm, la tige et le treillis étant munis d'un revêtement de protection contre la corrosion à long terme en oxyde de titane, caractérisé en ce que le treillis est enrobé en outre d'un matériau biocompatible biodégradable de faible module d'élasticité, ce matériau biocompatible biodégradable étant soit un copolymère d'acide lactique et d'acide glycolique, soit du collagène.

2. Tige selon la revendication 1, caractérisée en ce que le matériau biocompatible biodégradable est additionné de phosphate tricalcique.

3. Tige selon l'une des revendications 1 ou 2, caractérisée en ce que l'enrobage de matériau biocompatible biodégradable dépasse l'enveloppe du treillis de 0,3 à 1,5 mm.

4. Tige selon l'une des revendications précédentes, pour fixation sans ciment dans la partie supérieure du fémur, caractérisée en ce que le treillis lui est fixé au moins en partie par l'intermédiaire de minces bandes transversales (16) en titane ou alliage de titane, disposées à l'extérieur du treillis métallique et soudées aux surfaces latérales sensiblement planes de la tige sous le rebord d'appui (2) sur la partie supérieure du fémur après résection de sa tête, ainsi que par un point de soudure (18) à la pointe inférieure de la tige.

5. Tige selon l'une des revendications précédentes, pour fixation sans ciment dans la partie supérieure du fémur, caractérisée en ce que la tige est munie sur deux de ses faces opposées d'épaulements (6, 7, 8) disposés dans des plans perpendiculaires à son axe, sur toute sa hauteur jusqu'au rebord (2) d'appui sur la partie supérieure du fémur après résection de sa tête.

**Claims**

1. A stem (1) of titanium or titanium alloy for an articulated prothesis, conceived to be fixed without cement in a long bone surrounded by a titanium wire lattice (14) with a mesh size of at least 0,3 mm, said stem and said lattice being supplied with a protective coating of titanium oxide against long-term corrosion, characterized in that the lattice is further coated with a biodegradable and biocompatible material having a low modulus of elasticity, said biodegradable and biocompatible material being either a copolymer of lactic acid and glycolic acid or collagen.

2. A stem according to claim 1, characterized in that tricalcium phosphate is added to the biodegradable and biocompatible material.

3. A stem according to one of claims 1 or 2, characterized in that the coating of the biodegradable and biocompatible material extends 0,3 to 1,5 mm beyond the envelope of the lattice.

4. A stem according to one of the preceding claims, conceived to be fixed without cement in the upper part of the femur, characterized in that the lattice is at least partially attached to it by means of thin transverse strips (16) of titanium or titanium alloy disposed outside the metal lattice and welded to the substantially plane lateral surfaces of the stem below the support edge (2) which bears on the upper part of the femur after removal of its head, and by means of a spot weld (18) at the bottom of the stem.

5. A stem according to one of the preceding claims, conceived to be fixed without cement in the upper part of the femur, characterized in that the stem has shoulders (6, 7, 8) on two opposite sides disposed in planes perpendicular to its axis, extending over its entire height up to the support edge (2) which bears on said upper part of the femur after removal of its head.

**Patentansprüche**

1. Schaft (1) aus Titan oder einer Titanlegierung für eine Gelenkprothese zur zementfreien Befestigung in einem langen Knochen, umgeben von einem Gitter (14) aus Titandraht mit einer Maschenweite von mindestens 0,3 mm, wobei der Schaft und das Gitter mit einer Langzeit-Korrosionsschutzschicht aus Titanoxid umgeben sind, dadurch gekennzeichnet, daß das Gitter außerdem von einem biokompatiblen, bio-abbaubaren Material mit geringem Elastizitätsmodul umgeben ist, wobei dieses biokompatible, bioabbaubare Material entweder ein Copolymer von Milchsäure und Glycolsäure oder ein Collagen ist.

2. Schaft nach Anspruch 1, dadurch gekennzeichnet, daß dem biokompatiblen, bio-abbaubaren Material Tricalcium-Phosphat zugesetzt wird.

3. Schaft nach einem der Ansprüche 1 oder 2, dadurch gekennzeichnet, daß die Umhüllung mit dem biokompatiblen, bio-abbaubaren Materials um 0,3 bis 1,5 mm über die Hüllfläche des Gitters vorsteht.

4. Schaft nach einem der vorstehenden Ansprüche zur zementfreien Befestigung im oberen Teil des Oberschenkelknochens, dadurch gekennzeichnet, daß das Gitter an ihm mindestens teilweise über dünne Querbänder (16) aus Titan oder einer Titanlegierung, die außerhalb des Metallgitters angeordnet und an die im wesentlichen ebenen Seitenflächen des Schafts unter dem Rand (2) zur Abstützung auf dem oberen Teil des Oberschenkelknochens nach dessen Amputation angelötet sind, sowie über einen Lötpunkt (18) an der unteren Spitze des Schaftes befestigt ist.

5. Schaft nach einem der vorhergehenden Ansprüche für eine zementfreie Befestigung im oberen Teil des Oberschenkelknochens, dadurch gekennzeichnet, daß der Schaft auf zwei einander entgegengesetzten Seiten mit Vorsprüngen (6, 7, 8) versehen ist, die in zu seiner Achse querliegenden Ebenen angeordnet sind und sich über seine ganze Höhe bis zum Rand (2) zur Abstützung auf dem oberen Teil des Oberschenkelknochens nach Amputation seines Kopfes erstrecken.

# FIG.1

# FIG.2

# FIG.3